# EUROPEAN PATENT APPLICATION

(11) **EP 4 541 443 A1**
(43) Date of publication of application: **23.04.2025**
(21) Application number: 24202061.8
(22) Date of filing: 23.09.2024
(51) Int. Cl.: B01D 3/14

(54) **SEPARATION METHOD FOR A REACTION PRODUCT OF OLEFIN OLIGOMERIZATION, AND METHOD AND DEVICE FOR PREPARING ETHYLENE OLIGOMER UTILIZING THE SEPARATION METHOD**

(30) Priority: 17.10.2023 KR 20230138503
(71) Applicant: Hanwha TotalEnergies Petrochemical Co., Ltd., Seosan-si, Chungcheongnam-do 31900 (KR)
(72) Inventor: SOHN, Young Hoon, 31900 Seosan-si (KR); HONG, Won Jong, 31900 Seosan-si (KR); KO, Min Su, 31900 Seosan-si (KR); OH, Sang Joon, 31900 Seosan-si (KR); KANG, Ki Joon, 44992 Ulsan (KR); HARVIANTO, Gregorius Rionugroho, 44992 Ulsan (KR)
(74) Representative: Schrell, Andreas

(57) **Abstract**

The present invention relates to a separation method for a reaction product of olefin oligomerization, and a method and a device for preparing ethylene oligomer utilizing the separation method, and more specifically, a method capable of separating a mixture, which was generated after oligomerization reaction of ethylene and comprises a solvent and C8 or higher linear alpha olefins, into the solvent, C8 linear alpha olefin, and C10 or higher linear alpha olefin even by one-time treatment, and a method and a device for preparing ethylene oligomer utilizing the separation method.

## Description

### [TECHNICAL FIELD]

The present invention relates to a separation method for a reaction product of olefin oligomerization, and a method and a device for preparing ethylene oligomer utilizing the separation method, and more specifically, a method capable of separating a mixture, which was generated after oligomerization reaction of ethylene and comprises a solvent and C8 or higher linear alpha olefins, into the solvent, C8 linear alpha olefin, and C10 or higher linear alpha olefin even by one-time treatment, and a method and a device for preparing ethylene oligomer utilizing the separation method.

### [BACKGROUND ART]

Linear alpha olefins (LAO)-which are also referred to as linear alpha alkenes, linear terminal olefins, linear terminal alkenes, or normal alpha olefins-are mainly used as raw materials for various chemical processes, for example, such as comonomers in polymerization processes, precursors in oxidative production processes of aldehydes and/or carboxylic acids, starting materials in production processes of higher olefins, etc., and they are also directly utilized as raw material component for surfactants, lubricants, detergents, etc. In particular, among linear alpha olefins, 1-hexene and 1-octene are substances used in large quantities as comonomers in polymerization production of polyolefins such as polyethylene, and demand for them is steadily increasing as the production of polyolefins using homogeneous metallocene catalyst increases.

Linear alpha olefins are mainly synthesized through the oligomerization process of ethylene using Ziegler-type catalyst, and the product of such oligomerization process of ethylene is typically a (full-range) mixture of C4 or higher linear alpha olefins having an even number of carbon atoms (e.g., 1-butene, 1-hexene, 1-octene, 1-decene, etc.).

The product stream of such oligomerization process of ethylene comprises a plurality of linear alpha olefins, unreacted ethylene and a solvent inert to the oligomerization reaction, and these are separated into individual linear alpha olefins, unreacted ethylene and solvent through subsequent multiple distillation processes, and the separated ethylene and solvent are recovered for reuse.

By the way, in case of separating C8 linear alpha olefin, i.e., 1-octene which is the key material, from the reaction product of ethylene oligomerization process using C7 saturated hydrocarbons such as methylcyclohexane, heptane, etc. as inert solvent, the amount of solvent to be separated is very large, and the methylcyclohexane, heptane, etc. are lighter than 1-octene, and thus in the existing processes, the solvent is heated in a solvent distillation column and discharged from the overhead of the column, and the C8 or higher linear alpha olefins are discharged from the bottom of the column, and then these C8 or higher linear alpha olefins are fed into a subsequent distillation column and heated to recover 1-octene through the overhead, and C10 or higher linear alpha olefins are discharged from the bottom, thereby separating 1-octene.

However, such an existing separation method is disadvantageous in terms of process economics due to the use of excessive energy for separation by which costs for operation increase, and the requirement for multiple distillation columns by which costs for initial equipment investment and equipment maintenance increase, and in addition, the equipment becomes complicated and thus the stability and efficiency of the process become poor, and as a result, the quality and yield of the final product become unstable.

### [CONTENTS OF THE INVENTION]

### [PROBLEMS TO BE SOLVED]

The purpose of the present invention is to provide a method capable of separating a mixture, which was generated after oligomerization reaction of ethylene and comprises a solvent and C8 or higher linear alpha olefins, into the solvent, C8 linear alpha olefin, and C10 or higher linear alpha olefins in more economical and simpler manner, and a method and a device for preparing ethylene oligomer utilizing the separation method.

### [TECHNICAL MEANS]

The first aspect of the present invention relates to a method for separating a mixture comprising a solvent and C8 or higher linear alpha olefins, the method comprising:
distilling a mixture comprising a solvent having a boiling point lower than C8 linear alpha olefin, C8 linear alpha olefin, and C10 or higher linear alpha olefin in a dividing wall column, to discharge the solvent from the overhead of the dividing wall column, discharge the C8 linear alpha olefin from the first side of the dividing wall column, and discharge the C10 or higher linear alpha olefin from the bottom or a lower side than the first side of the dividing wall column.

In an embodiment, the solvent having a boiling point lower than C8 linear alpha olefin for use may be an aliphatic hydrocarbon solvent having 5 to 20 carbon atoms, an aromatic hydrocarbon solvent having 6 to 20 carbon atoms, or a mixture thereof, and more concretely, it may be selected from the group consisting of benzene, toluene, xylene, chlorobenzene, dichlorobenzene, pentane, hexane, heptane, methylcyclohexane, cyclohexane and combinations thereof.

In an embodiment, the mixture may comprise, based on total 100 % by weight of the mixture, 50 to 95 % by weight of the solvent having a boiling point lower than C8 linear alpha olefin, 4 to 40 % by weight of the C8 linear alpha olefin, and 0.5 to 10 % by weight of the C10 or higher linear alpha olefin.

In an embodiment, the distillation in the dividing wall column may be conducted at a temperature of 40°C to 250°C and under a pressure of 0.1 bar to 15 bar.

In an embodiment, the C10 or higher linear alpha olefin discharged from the dividing wall column is distilled additionally.

The second aspect of the present invention relates to a method for preparing ethylene oligomer, the method comprising the steps of:
(1) conducting ethylene oligomerization reaction in a solvent having a boiling point lower than C8 linear alpha olefin;
(2) separating from the resulting reaction mixture of step (1), a mixture comprising the solvent and C8 or higher linear alpha olefins; and
(3) distilling the mixture separated in step (2) comprising the solvent and C8 or higher linear alpha olefins in a dividing wall column, to discharge the solvent from the overhead of the dividing wall column, discharge C8 linear alpha olefin from the first side of the dividing wall column, and discharge C10 or higher linear alpha olefin from the bottom or a lower side than the first side of the dividing wall column.

In an embodiment, the solvent having a boiling point lower than C8 linear alpha olefin for use may be an aliphatic hydrocarbon solvent having 5 to 20 carbon atoms, an aromatic hydrocarbon solvent having 6 to 20 carbon atoms, or a mixture thereof, and more concretely, it may be selected from the group consisting of benzene, toluene, xylene, chlorobenzene, dichlorobenzene, pentane, hexane, heptane, methylcyclohexane, cyclohexane and combinations thereof.

In an embodiment, additionally in step (2), unreacted ethylene is recovered for reuse in the oligomerization reaction of ethylene in step (1).

In an embodiment, additionally in step (2), C6 or lower linear alpha olefin is separated from the resulting reaction mixture of step (1).

In an embodiment, the C6 or lower linear alpha olefin separated in step (2) is distilled additionally.

In an embodiment, the mixture distilled in the dividing wall column in step (3) may comprise, based on total 100 % by weight of the mixture, 50 to 95 % by weight of the solvent having a boiling point lower than C8 linear alpha olefin, 4 to 40 % by weight of the C8 linear alpha olefin, and 0.5 to 10 % by weight of the C10 or higher linear alpha olefin.

In an embodiment, the distillation in the dividing wall column in step (3) may be conducted at a temperature of 40°C to 250°C and under a pressure of 0. 1 bar to 15 bar.

In an embodiment, the solvent discharged from the dividing wall column in step (3) is recovered for reuse in the oligomerization reaction of ethylene in step (1).

In an embodiment, the C10 or higher linear alpha olefin discharged from the dividing wall column in step (3) is distilled additionally.

The third aspect of the present invention relates to a device for preparing ethylene oligomer, the device comprising:
(a) an ethylene oligomerization reactor;
(b) a first distillation column in fluid communication with the bottom of the ethylene oligomerization reactor; and
(c) a dividing wall column in fluid communication with the bottom of the first distillation column,
wherein the device is operated in a manner that:
in the ethylene oligomerization reactor, ethylene oligomerization reaction is conducted in a solvent having a boiling point lower than C8 linear alpha olefin,
the resulting mixture of the ethylene oligomerization reaction is discharged from the bottom of the ethylene oligomerization reactor, and it is then fed into the first distillation column and distilled therein,
as a result of the distillation in the first distillation column, a mixture comprising the solvent and C8 or higher linear alpha olefins is discharged from the bottom of the first distillation column, and it is then fed into the dividing wall column and distilled therein, and
as a result of the distillation in the dividing wall column, the solvent is discharged from the overhead of the dividing wall column, C8 linear alpha olefin is discharged from the first side of the dividing wall column, and C10 or higher linear alpha olefin is discharged from the bottom or a lower side than the first side of the dividing wall column.

In an embodiment, the device for preparing ethylene oligomer further comprises an ethylene recovery line fluidly communicating the first distillation column and the ethylene oligomerization reactor.

In an embodiment, the device for preparing ethylene oligomer further comprises a device for distilling C6 or lower linear alpha olefin which is in fluid communication with the overhead of the first distillation column.

In an embodiment, the device for preparing ethylene oligomer further comprises a solvent recovery line fluidly communicating the dividing wall column and the ethylene oligomerization reactor.

In an embodiment, the device for preparing ethylene oligomer further comprises a device for distilling C10 or higher linear alpha olefin which is in fluid communication with the bottom of the dividing wall column.

### [EFFECT OF THE INVENTION]

According to the present invention, in ethylene oligomerization, the amount of energy use can be significantly reduced as compared with the existing traditional separation processes while producing the product with the same specifications as those of the existing processes, and since the equipment becomes simplified, costs for initial equipment investment and equipment maintenance can be reduced, resulting in advantage in terms of economy and also the effect of improving the stability and efficiency of the process.

### [BRIEF EXPLANATION OF THE DRAWINGS]

Figure 1 corresponds to the comparative example of the present invention and schematically shows an existing general process for ethylene oligomerization and product separation.
Figure 2 corresponds to the example of the present invention and schematically shows a process for ethylene oligomerization and product separation according to an embodiment of the present invention.
Figure 3 schematically shows an embodiment of a process for further distilling C10 or higher linear alpha olefin (C10+) discharged from the process shown in Figure 2.
Figure 4 schematically shows another embodiment of a process for further distilling C10 or higher linear alpha olefin (C10+) discharged from the process shown in Figure 2.

### [CONCRETE MODE FOR CARRYING OUT THE INVENTION]

The present invention is explained in more detail below.

The term "linear alpha olefin" (LAO) as used herein refers to a linear alkene compound having a terminal unsaturated double bond.

The term "oligomer of ethylene" or "ethylene oligomer" as used herein refers to a linear alpha olefin having an even number of carbon atoms of 4 or more (chemical formula C₂ₙH₄ₙ, where n is a positive integer of 2 or more) which may be, for example, 1-butene, 1-hexene, 1-octene or 1-decene, etc., but it is not limited thereto.

As the symbols used herein, "Cx" represents a linear alpha olefin (or ethylene oligomer) having x carbon atoms, "Cx-" represents a linear alpha olefin having x or less carbon atoms, and "Cx+" represents a linear alpha olefin having x or more carbon atoms, and for example, "C8" is a linear alpha olefin having 8 carbon atoms, i.e., 1-octene, "C8-" is a linear alpha olefin having 8 or less carbon atoms, i.e., including 1-butene, 1-hexene and 1-octene, and "C8+" is a linear alpha olefin having 8 or more carbon atoms, i.e., including 1-octene, 1-decene, 1-dodecene and the like.

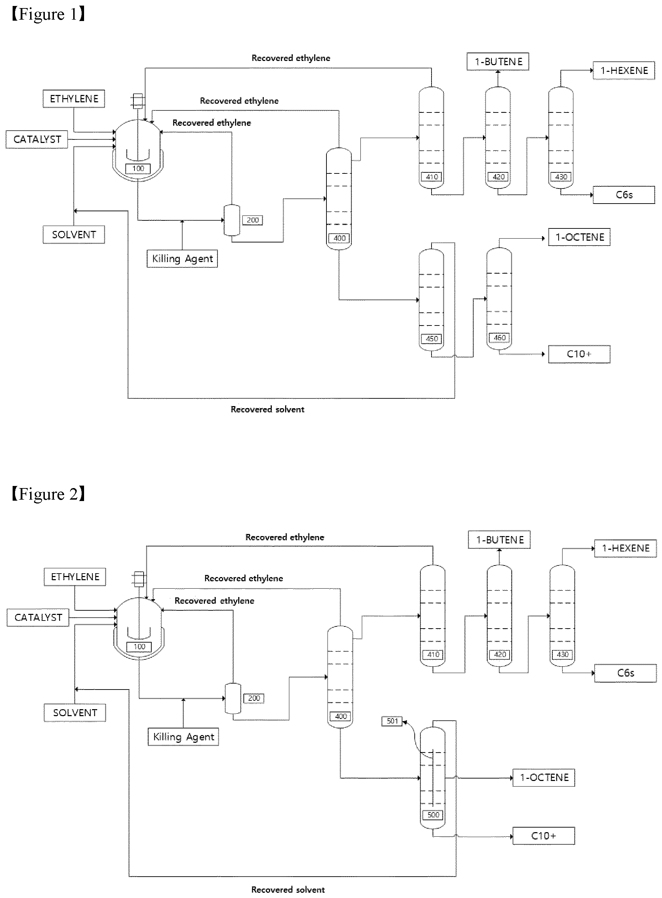

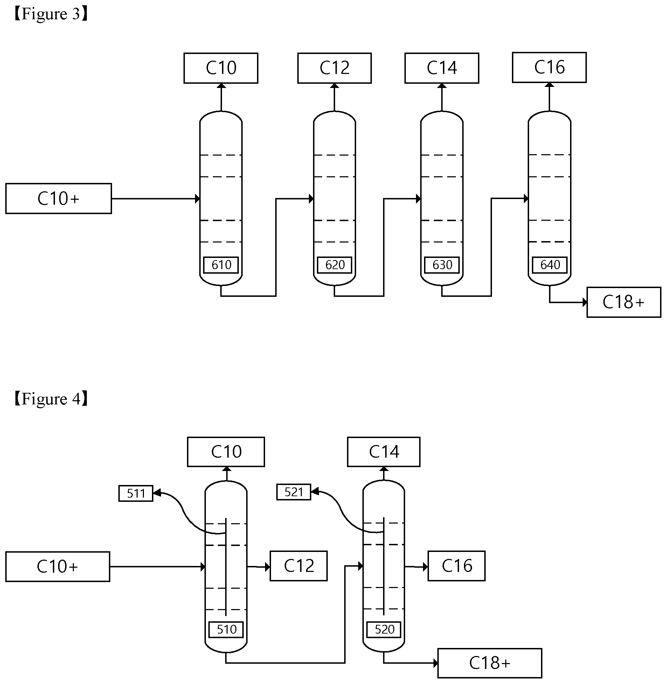

In the present invention, the "dividing wall column" (DWC) comprises one or more dividing walls inside the distillation column, and the space inside the distillation column is divided into two or more spaces by the dividing walls. If a mixture is distilled in the dividing wall column, three or more distillation product streams are discharged wherein the lightest stream (lowest boiling point) is discharged from the overhead of the dividing wall column, the heaviest stream (highest boiling point) is discharged from the bottom of the dividing wall column, and the intermediate stream(s) is(are) discharged through one or more sides disposed on the wall surface of the dividing wall column. If there is one dividing wall, it is called a single dividing wall column, and in this case, three distillation product streams (overhead/side/bottom) are discharged. If there are two or more dividing walls, it is called a multiple dividing wall column, and in this case, four or more distillation product streams are discharged. For example, in case of a double dividing wall column with two dividing walls, four distillation product streams (overhead/first side/second side/bottom) are discharged. Single dividing wall columns (500, 510, 520) are illustrated in Figures 2 and 4 of the present specification.

In an embodiment of the present invention, the solvent having a boiling point lower than C8 linear alpha olefin may be an aliphatic hydrocarbon solvent having 5 to 20 carbon atoms, an aromatic hydrocarbon solvent having 6 to 20 carbon atoms, or a mixture thereof, and more concretely, it may be selected from the group consisting of benzene, toluene, xylene, chlorobenzene, dichlorobenzene, pentane, hexane, heptane, methylcyclohexane, cyclohexane and combinations thereof, and still more concretely, it may be selected from the group consisting of methylcyclohexane, cyclohexane, heptane and combinations thereof, and preferably it may be methylcyclohexane.

In an embodiment of the present invention, the mixture distilled in the dividing wall column may comprise, based on total 100 % by weight of the mixture, 50 to 95 % by weight of the solvent having a boiling point lower than C8 linear alpha olefin, 4 to 40 % by weight of C8 linear alpha olefin, and 0.5 to 10 % by weight of C10 or higher linear alpha olefin.

If the amount of the solvent in total 100 % by weight of the mixture distilled in the dividing wall column is less than 50 % by weight, there may be a problem of blocking lines due to excessive oligomerization reaction and polymer generation thereby, and to the contrary, if it is greater than 95 % by weight, there may be a problem of decreasing productivity due to underproduction of C8 and increase of the ratio of C4 and C6.

More concretely, the amount of the solvent in total 100 % by weight of the mixture distilled in the dividing wall column may be 55 % by weight or more, 60 % by weight or more, 65 % by weight or more, 70 % by weight or more, 75 % by weight or more, or 80 % by weight or more, and also may be 94 % by weight or less, 93 % by weight or less, 92 % by weight or less, 91 % by weight or less, or 90 % by weight or less, but it is not limited thereto.

Also, more concretely the amount of the C8 linear alpha olefin in total 100 % by weight of the mixture distilled in the dividing wall column may be 5 % by weight or more, 6 % by weight or more, 7 % by weight or more, 8 % by weight or more, or 9 % by weight or more, and also may be 35 % by weight or less, 30 % by weight or less, 25 % by weight or less, 20 % by weight or less, or 15 % by weight or less, but it is not limited thereto.

Also, more concretely the amount of the C10 or higher linear alpha olefin in total 100 % by weight of the mixture distilled in the dividing wall column may be 0.6 % by weight or more, 0.7 % by weight or more, 0.8 % by weight or more, 0.9 % by weight or more, or 1 % by weight or more, and also may be 9 % by weight or less, 8 % by weight or less, 7 % by weight or less, 6 % by weight or less, or 5 % by weight or less, but it is not limited thereto.

In an embodiment of the present invention, the distillation in the dividing wall column may be conducted at a temperature of 40°C to 250°C and under a pressure of 0.1 bar to 15 bar.

If the temperature during the distillation in the dividing wall column is lower than 40°C, there may be a problem that all components are not distilled but discharged through the bottom and the overhead of the distillation column is dried up, and to the contrary, if it is higher than 250°C, there may be a problem that all components are distilled and discharged through the overhead and thus the bottom of the distillation column is dried up. In addition, if the pressure during the distillation in the dividing wall column is lower than 0.1 bar, there may be a problem that the boiling point of the fluid is lowered and thus the separation does not occur normally, and the purity of the solvent discharged from the overhead and recycled decreases and thus the reactivity is lowered and the purity of 1-octene is decreased, and to the contrary, if it is higher than 15 bar, there may be a problem that the boiling point of the fluid is elevated to 250°C or higher and thus the distillation does not occur normally, causing decrease of the production amount of 1-octene, loss of the solvent due to its discharge not from the overhead but from the side, and decrease of the purity of 1-octene discharged from the side.

More concretely, the temperature condition during the distillation in the dividing wall column may be 41°C or higher, 42°C or higher, 43°C or higher, 44°C or higher, 45°C or higher, 46°C or higher, 47°C or higher, 48°C or higher, 49°C or higher, or 50°C or higher, and also may be 250°C or lower, 240°C or lower, 230°C or lower, 220°C or lower, 210°C or lower, 200°C or lower, 190°C or lower, or 180°C or lower, but it is not limited thereto.

Also, more concretely the pressure condition during the distillation in the dividing wall column may be 0.15 bar or higher, 0.2 bar or higher, 0.25 bar or higher, or 0.3 bar or higher, and also may be 15 bar or lower, 14 bar or lower, 12 bar or lower, 10 bar or lower, 8 bar or lower, 6 bar or lower, 5 bar or lower, 4 bar or lower, 3 bar or lower, 2 bar or lower, or 1 bar or lower, but it is not limited thereto.

In an embodiment of the present invention, at the overhead of the dividing wall column, the temperature condition may be 40°C or higher, 45°C or higher, or 50°C or higher, and also may be 80°C or lower, 70°C or lower, or 60°C or lower, but it is not limited thereto, and the pressure condition may be 0.1 bar or higher, 0.15 bar or higher, or 0.2 bar or higher, and also may be 1 bar or lower, 0.85 bar or lower, 0.5 bar or lower, or 0.4 bar or lower, but it is not limited thereto.

In an embodiment of the present invention, at the side of the dividing wall column, the temperature condition may be 60°C or higher, 70°C or higher, or 80°C or higher, and also may be 130°C or lower, 120°C or lower, 110°C or lower, or 100°C or lower, but it is not limited thereto, and the pressure condition may be 0.3 bar or higher, 0.35 bar or higher, or 0.4 bar or higher, and also may be 1.2 bar or lower, 1 bar or lower, 0.8 bar or lower, or 0.6 bar or lower, but it is not limited thereto.

In an embodiment of the present invention, at the bottom of the dividing wall column, the temperature condition may be 130°C or higher, 140°C or higher, or 150°C or higher, and also may be 250°C or lower, 240°C or lower, 230°C or lower, 220°C or lower, 200°C or lower, or 180°C or lower, but it is not limited thereto, and the pressure condition may be 0.4 bar or higher, 0.45 bar or higher, or 0.5 bar or higher, and also may be 15 bar or lower, 14 bar or lower, 12 bar or lower, 10 bar or lower, 8 bar or lower, 6 bar or lower, 5 bar or lower, 4 bar or lower, 3 bar or lower, 2 bar or lower, or 1 bar or lower, but it is not limited thereto.

In an embodiment of the present invention, the C10 or higher linear alpha olefin discharged from the dividing wall column is distilled additionally.

In an embodiment, the C10 or higher linear alpha olefin may be distilled additionally by passing through one or more distillation columns sequentially. For example, as illustrated in Figure 3 of the present specification, the C10 or higher linear alpha olefin stream (C10+) discharged from the dividing wall column is distilled by passing through four distillation columns (610, 620, 630, 640) sequentially, and thereby C10, C12, C14 and C16 are collected from the overhead of each distillation column, respectively, and the final residue C18+ is discharged from the bottom of the final distillation column (640). Alternatively, as illustrated in Figure 4 of the present specification, C10+ discharged from the dividing wall column is distilled by passing through two single dividing wall columns (510, 520) sequentially, and thereby C10 and C12 are collected from the overhead and side of the distillation column (510), respectively, C14 and C16 are collected from the overhead and side of the distillation column (520), respectively, and the final residue C18+ is discharged from the bottom of the final distillation column (520).

In the method for preparing ethylene oligomer which is the second aspect of the present invention, in step (1), ethylene oligomerization reaction is conducted in a solvent having a boiling point lower than C8 linear alpha olefin.

The solvent having a boiling point lower than C8 linear alpha olefin is the same as explained above, and the ethylene oligomerization reaction can be conducted in the presence of catalyst according to a known method.

For the above catalyst, any catalyst known for oligomerization reaction of ethylene can be used without particular limitation, and more concretely, any known chromium catalyst can be used as is or with appropriate modification, and for example, a chromium compound of the following chemical formula 1 disclosed in Korean Laid-open Patent Publication No. 10-2022-0087986 can be used as the catalyst, but it is not limited thereto. 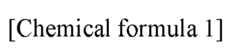 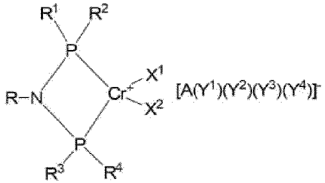 In the above chemical formula 1,
R is C1-C60 alkyl or C6-C60 aryl;
each of R¹ to R⁴ is independently C1-C60 alkyl or C6-C60 aryl;
each of X¹ and X² is independently C1-C30 hydrocarbyl comprising one or more selected from halogen, C1-C30 alkyl, C1-C30 alkylcarboxylate, acetylacetonate, ether and amino;
A is boron or aluminum;
each of Y¹ to Y⁴ is independently C6-C60 aryl substituted with fluorine, C6-C60 aryloxy substituted with fluorine, or C6-C60 alkoxy substituted with fluorine; and
the alkyl or aryl of the above R and the alkyl or aryl of the above R¹ to R⁴ may be further substituted by one or more selected from C1-C30 alkyl, C6-C30 aryl, C1-C30 alkoxy, mono-C1-C30 alkylamino, di-C1-C30 alkylamino, tri-C1-C30 alkylamino, mono-C6-C30 arylamino, di-C6-C30 arylamino, tri-C6-C30 arylamino, mono-C1-C30 alkylsilyl, di-C1-C30 alkylsilyl, tri-C1-C30 alkylsilyl, mono-C6-C30 arylsilyl, di-C6-C30 arylsilyl and tri-C6-C30 arylsilyl.

Also, there is no particular limitation in the method of oligomerizing ethylene, and for example, as disclosed in Korean Laid-open Patent Publication No. 10-2022-0087986, the oligomerization of ethylene can be conducted under the conditions of ethylene pressure of 0 to 100 kgf/cm² and reaction temperature of 0 to 150°C, more concretely, under the conditions of ethylene pressure of 20 to 80 kgf/cm² and reaction temperature of 20 to 120°C, and still more concretely, under the conditions of ethylene pressure of 30 to 60 kgf/cm² and reaction temperature of 40 to 100°C, but it is not limited thereto.

The entire contents described in Korean Laid-open Patent Publication No. 10-2022-0087986, including the chromium compound of the above chemical formula 1 and the ethylene oligomerization conditions, are utilized herein as a reference for the ethylene oligomerization reaction, unless otherwise specified.

In an embodiment of the present invention, immediately after the ethylene oligomerization reaction of step (1), a killing agent is added to the resulting reaction mixture to prevent further reaction in the subsequent separation process. The killing agent for use may be one or more selected from the group consisting of 1-butanol, 2-butanol, iso-butanol, sec-butanol, t-butanol, 1-hexanol, 2-hexanol, 3-hexanol, 1-heptanol, 2-heptanol, 3-heptanol, 4-heptanol, 1-octanol, 2-octanol, 3-octanol, 4-octanol, 2-ethyl-1-hexanol, 2-methyl-3-heptanol, 1-decanol, 2-decanol, 3-decanol, 4-decanol, 5-decanol, 1-undecanol, 2-undecanol, 7-methyl-2-decanol, 1-dodecanol, 2-dodecanol, 2-ethyl-1-decanol and mixtures thereof, but it is not limited thereto.

Preferably, in order to prevent the killing agent from being recovered together during solvent recovery, one or more selected from the group consisting of 1-octanol, 1-decanol, 1-dodecanol, 2-ethyl-1-hexanol, 2-ethyl-1-octanol, 2-methyl-3-heptanol, 1-decanol, 2-decanol, 3-decanol, 4-decanol, 5-decanol, 1-undecanol, 2-undecanol, 7-methyl-2-decanol, 1-dodecanol, 2-dodecanol, 2-ethyl-1-decanol and mixtures thereof-which have a boiling point higher than 170°C that is the boiling point of 1-decene-may be used.

In an embodiment of the present invention, the resulting reaction mixture of step (1) may be passed through a gas-liquid separator to recover unreacted ethylene before entering into step (2), and the unreacted ethylene recovered as such is reused in the ethylene oligomerization reaction of step (1).

In an embodiment of the present invention, additionally in step (2), C6 or lower linear alpha olefin is separated from the resulting reaction mixture of step (1).

In an embodiment of the present invention, the C6 or lower linear alpha olefin separated in step (2) is distilled additionally.

In an embodiment, the C6 or lower linear alpha olefin may be distilled additionally by passing through one or more distillation columns sequentially. For example, as illustrated in Figures 1 and 2 of the present specification, the C6 or lower linear alpha olefin stream discharged from the overhead of the distillation column (400) is distilled by passing through three distillation columns (410, 420, 430) sequentially, and thereby ethylene, 1-butene, and 1-hexene are collected from the overhead of each distillation column, respectively, and the final residue (C6s, substances with a higher boiling point than 1-hexene) is discharged from the bottom of the final distillation column (430).

In an embodiment, since the above-explained recovered ethylene stream may contain not only ethylene but also hydrogen, 1-butene, 1-hexene, solvent, etc., a constitution for preventing increase of impurity contents in the recovered material by discharging a portion of the recovered ethylene stream as off-gas may be added.

In the method for preparing ethylene oligomer of the present invention, in step (3), the mixture separated in step (2) comprising the solvent and C8 or higher linear alpha olefins is distilled in a dividing wall column, and thereby the solvent is discharged from the overhead of the dividing wall column, C8 linear alpha olefin is discharged from the first side of the dividing wall column, and C10 or higher linear alpha olefin is discharged from the bottom or a lower side than the first side of the dividing wall column.

The concrete composition of the mixture fed into the dividing wall column, and the concrete constitution of the dividing wall column and distillation conditions therein are the same as explained above.

In an embodiment, the solvent discharged from the dividing wall column in step (3) is recovered for reuse in the oligomerization reaction of ethylene in step (1). At this time, the killing agent should not be recovered.

In an embodiment, the C10 or higher linear alpha olefin discharged from the dividing wall column in step (3) is distilled additionally. The additional distillation of the C10 or higher linear alpha olefin is the same as explained above.

According to an embodiment, in the procedure of recovering raw materials and solvent of the ethylene oligomerization reaction, the recovered materials may be cooled using a heat exchanger.

According to an embodiment, before being fed into the gas-liquid separator, the resulting mixture of ethylene oligomerization reaction may be passed through a filter to separate polymers therefrom.

According to an embodiment, the C10 or higher linear alpha olefin discharged from the dividing wall column may be treated using a drum flaker or the like to separate low molecular weight polymers therefrom.

According to an embodiment, the raw materials and solvent for ethylene oligomerization reaction may be treated using an adsorption column before introducing them into the reactor so that catalyst poisons such as moisture and oxygen can be removed therefrom. Such a pretreatment procedure can be carried out generally through an adsorption process.

The ethylene oligomerization reactor (a) comprised in the device for preparing ethylene oligomer, which is the third aspect of the present invention, may be a conventional reactor, for example, such as a stirred reactor, a tubular reactor or a continuous stirred tank reactor, etc. and may have an ethylene injection part, a catalyst injection part and a solvent injection part, and may further have a recovered ethylene injection part. Two or more ethylene oligomerization reactors may be installed in a parallel configuration.

The first distillation column (b) comprised in the device for preparing ethylene oligomer of the present invention may be a conventional distillation column, and it is in fluid communication with the bottom of the ethylene oligomerization reactor and a gas-liquid separator may be placed therebetween. Two or more gas-liquid separators may be installed in a parallel or serial configuration.

In an embodiment, between the first distillation column and the ethylene oligomerization reactor, an ethylene recovery line fluidly communicating them may exist.

In an embodiment, the overhead of the first distillation column may be in fluid communication with an additional device for distilling C6 or lower linear alpha olefin, and such an additional device may include a plurality of distillation columns such as the distillation column configurations (410, 420, 430) illustrated in Figures 1 and 2 of the present specification, but it is not limited thereto.

The dividing wall column (c) comprised in the device for preparing ethylene oligomer of the present invention is in fluid communication with the bottom of the first distillation column, and it separates the mixture comprising the solvent and C8 or higher linear alpha olefins from the bottom of the first distillation column into the solvent, C8 linear alpha olefin, and C10 or higher linear alpha olefin, respectively. The concrete composition of the mixture fed into the dividing wall column, and the concrete constitution of the dividing wall column and distillation conditions therein are the same as explained above.

In an embodiment, between the dividing wall column and the ethylene oligomerization reactor, an ethylene recovery line fluidly communicating them may exist.

In an embodiment, the bottom of the dividing wall column may be in fluid communication with an additional device for distilling C10 or higher linear alpha olefin, and such an additional device may include a plurality of distillation columns such as the distillation column configurations (610, 620, 630, 640) illustrated in Figure 3 or the distillation column configurations (510, 520) illustrated in Figure 4 of the present specification, but it is not limited thereto.

The following is more specific explanation with reference to the drawings, but the scope of the present invention is not limited by these drawings in any manner.

Figure 1 schematically shows an existing general process for ethylene oligomerization and product separation, and in this process, ethylene as raw material, a catalyst and a solvent are fed into an oligomerization reactor (100) and the reaction is conducted; then the reaction product passes through a gas-liquid separator (200) and is fed into a first distillation column (400) and distilled therein, and separated into the overhead stream containing C6 or lower linear alpha olefin and the bottom stream containing C8 or higher linear alpha olefins and discharged; then the overhead stream of the first distillation column is distilled by passing through three distillation columns (410, 420, 430) sequentially, and thereby ethylene, 1-butene, and 1-hexene are collected from the overhead of each distillation column, respectively, and C6s (a substance with a higher boiling point than 1-hexene) is discharged from the bottom of the final distillation column (430); and the bottom stream of the first distillation column is distilled by passing through two distillation columns (450, 460) sequentially, and thereby the recovered solvent and 1-octene are collected from the overhead of each distillation column, respectively, and C10+ (C10 or higher linear alpha olefin stream) is discharged from the bottom of the final distillation column (460). The recovered solvent is reintroduced into the reactor (100), and also unreacted ethylene is recovered from the gas-liquid separator (200) and distillation columns (400, 410) and reintroduced into the reactor (100).

Figure 2 schematically shows a process for ethylene oligomerization and product separation according to an embodiment of the present invention, and this process is the same as that of Figure 1 in terms of the oligomerization reaction, distillation procedure in the first distillation column, and distillation procedure of the overhead stream of the first distillation column, but it is different from that of Figure 1 in that the bottom stream of the first distillation column is distilled in a single dividing wall column (500), and thereby the solvent is discharged from the overhead of the dividing wall column, the C8 linear alpha-olefin (1-octene) is discharged from the side of the dividing wall column, and the C10 or higher linear alpha olefin (C10+) is discharged from the bottom of the dividing wall column.

Figures 3 and 4 schematically show embodiments of a process for further distilling C10 or higher linear alpha olefin (C10+) discharged from the bottom of the dividing wall column (500) in the process shown in Figure 2, which are the same as explained above.

The present invention is explained in more detail through the following Examples. However, the scope of the present invention is not limited by the Examples in any manner.

### [EXAMPLES]

### Preparation Example: Ethylene oligomerization reaction and the first separation of the reaction product

According to the process shown in Figure 1, the ethylene oligomerization reaction and the first separation procedure of the reaction product were performed as follows.

In tubular reactor (100) with 1.2 m length and 2-inch diameter, an ethylene oligomerization reaction was conducted in the presence of a catalyst [(iPrN[P(C₆H₄-p-Si(nBu)₃)₂]₂)-CrCl₂]⁺[B(C₆F₅)₄]⁻ (1 µmol) and 200 µmol of triisobutylaluminum (Al/Cr ratio: 200) using methylcyclohexane as a solvent. The pressure of the reactant ethylene was 35 kgf/cm², and the temperature was 40°C. Thereafter, the resulting mixture of the oligomerization reaction was passed through a gas-liquid separator (200) and introduced into a first distillation column (400) and distilled therein.

As a result of gas chromatography (GC) analysis, the overhead stream discharged from the first distillation column contained 73.0 % by weight of ethylene, 2.4 % by weight of C4 linear alpha-olefin and 15.7 % by weight of C6 linear alpha-olefin, and the bottom stream discharged from the first distillation column contained 86.6 % by weight of methylcyclohexane solvent, 11.9 % by weight of C8 linear alpha-olefin and 1.4 % by weight of C 10 or higher linear alpha olefin.

### Comparative Example

According to the process shown in Figure 1, the overhead stream discharged from the first distillation column obtained in the above Preparation Example was distilled by passing through three distillation columns (410, 420, 430) sequentially, and thereby ethylene, 1-butene, and 1-hexene were collected from the overhead of each distillation column, respectively, and the bottom stream discharged from the first distillation column was distilled by passing through two distillation columns (450, 460) sequentially, and the solvent and 1-octene were collected from the overhead of each distillation column, respectively. For cooling the overhead stream of the distillation column, industrial water at a temperature not exceeding 40°C was used as cooling water, and a propylene cooler with operation capacity of -35°C was used.

The energy consumption (consumption during distillation and consumption during overhead stream cooling) in each distillation column was calculated, and the relative values, when the total energy consumption was set to 100, are shown in Table 1 below, and the purities and flow amount ratios of the obtained solvent and linear alpha-olefins are shown in Table 2 below.

**[Table 1]**

| Column No. | Facility name | Energy Consumption during distillation | Energy Consumption during cooling |
|---|---|---|---|
| 400 | Distillation column for low-boiling point substance separation | 25.0 | 7.6 |
| 410 | Distillation column for ethylene recovery | 0.9 | 0.6 |
| 420 | Distillation column for 1-butene separation | 0.1 | 0.4 |
| 430 | Distillation column for1-hexene separation | 3.5 | 3.6 |
| 450 | Distillation column for solvent recovery | 17.2 | 33.4 |
| 460 | Distillation column for 1-octene separation | 3.8 | 3.9 |
| Total Energy Consumption | | **50.5** | **49.5** |

**[Table 2]**

| Kind | Purity (wt%) | Flow amount ratio |
|---|---|---|
| 1-Butene | 99.98 | 0.2 |
| 1-Hexene | 99.70 | 2.1 |
| Methylcyclohexane solvent | 99.92 | 85.8 |
| 1-Octene | 98.47 | 11.9 |

### Example

According to the process shown in Figure 2, the overhead stream discharged from the first distillation column obtained in the above Preparation Example was distilled by passing through three distillation columns (410, 420, 430) sequentially, and thereby ethylene, 1-butene, and 1-hexene were collected from the overhead of each distillation column, respectively, and the bottom stream discharge from the first distillation column was passed through a dividing wall column (500), and therein the solvent was separated and collected from the overhead of the dividing wall column, C8 linear alpha-olefin (1-octene) was separated and collected from the side of the dividing wall column, and C10 or higher linear alpha olefin (C10+) was separated and collected from the bottom of the dividing wall column. The distillation in the dividing wall column was conducted within a temperature range of 52°C to 167°C and a pressure range of 0.33 to 0.53 bar, concretely under the conditions of 0.33 bar/52°C at the overhead, 0.45 bar/95°C at the side, and 0.53 bar/167°C at the bottom. For cooling the overhead stream of the distillation column, industrial water at a temperature not exceeding 40°C was used as cooling water, and if necessary, a propylene cooler with operation capacity of -35°C was used.

The energy consumption (consumption during distillation and consumption during overhead stream cooling) in each distillation column was calculated, and the relative values, when the total energy consumption was set to 100, are shown in Table 3 below, and the purities and flow amount ratios of the obtained solvent and linear alpha-olefins are shown in Table 4 below.

**[Table 3]**

| Column No. | Facility name | Energy Consumption during distillation | Energy Consumption during cooling |
|---|---|---|---|
| 400 | Distillation column for low-boiling point substance separation | 25.0 | 7.6 |
| 410 | Distillation column for ethylene recovery | 0.9 | 0.6 |
| 420 | Distillation column for 1-butene separation | 0.1 | 0.4 |
| 430 | Distillation column for1-hexene separation | 3.5 | 3.6 |
| 500 | Dividing wall column | 12.3 | 28.5 |
| Total Energy Consumption | | **41.8** | **40.7** |

**[Table 4]**

| Kind | Purity (wt%) | Flow amount ratio |
|---|---|---|
| 1-Butene | 99.98 | 0.2 |
| 1-Hexene | 99.70 | 2.1 |
| Methylcyclohexane solvent | 99.92 | 85.8 |
| 1-Octene | 98.47 | 11.9 |

As confirmed from the results in the above Tables 1 to 4, in the Example of the present invention, it was possible to produce a linear alpha-olefin product with the same specifications as the Comparative Example, which is the existing process, while significantly reducing energy consumption compared with the Comparative Example (i.e., the total energy consumption was relatively reduced by 17.5% (=100-82.5)).

### [EXPLANATION OF THE REFERENCE NUMERALS]

100: Oligomerization reactor
200: Gas-liquid separator
400, 410, 420, 430, 450, 460, 610, 620, 630, 640: Distillation column
500, 510, 520: Dividing wall column
501, 511, 521: Dividing wall

## Claims

1. A method for separating a mixture comprising a solvent and C8 or higher linear alpha olefins, the method comprising:
distilling a mixture comprising a solvent having a boiling point lower than C8 linear alpha olefin, C8 linear alpha olefin, and C10 or higher linear alpha olefin in a dividing wall column, to discharge the solvent from the overhead of the dividing wall column, discharge the C8 linear alpha olefin from the first side of the dividing wall column, and discharge the C10 or higher linear alpha olefin from the bottom or a lower side than the first side of the dividing wall column.

2. The method of claim 1, wherein the solvent having a boiling point lower than C8 linear alpha olefin is selected from the group consisting of benzene, toluene, xylene, chlorobenzene, dichlorobenzene, pentane, hexane, heptane, methylcyclohexane, cyclohexane and combinations thereof.

3. The method of claim 1, wherein the mixture comprises, based on total 100 % by weight of the mixture, 50 to 95 % by weight of the solvent having a boiling point lower than C8 linear alpha olefin, 4 to 40 % by weight of the C8 linear alpha olefin, and 0.5 to 10 % by weight of the C10 or higher linear alpha olefin.

4. The method of claim 1, wherein the distillation in the dividing wall column is conducted at a temperature of 40°C to 250°C and under a pressure of 0. 1 bar to 15 bar.

5. The method of claim 1, wherein the C10 or higher linear alpha olefin discharged from the dividing wall column is distilled additionally.

6. A method for preparing ethylene oligomer, the method comprising the steps of:
(1) conducting ethylene oligomerization reaction in a solvent having a boiling point lower than C8 linear alpha olefin;
(2) separating from the resulting reaction mixture of step (1), a mixture comprising the solvent and C8 or higher linear alpha olefins; and
(3) distilling the mixture separated in step (2) comprising the solvent and C8 or higher linear alpha olefins in a dividing wall column, to discharge the solvent from the overhead of the dividing wall column, discharge C8 linear alpha olefin from the first side of the dividing wall column, and discharge C10 or higher linear alpha olefin from the bottom or a lower side than the first side of the dividing wall column.

7. The method of claim 6, wherein the solvent having a boiling point lower than C8 linear alpha olefin is selected from the group consisting of benzene, toluene, xylene, chlorobenzene, dichlorobenzene, pentane, hexane, heptane, methylcyclohexane, cyclohexane and combinations thereof.

8. The method of claim 6, wherein, additionally in step (2), unreacted ethylene is recovered for reuse in the oligomerization reaction of ethylene in step (1).

9. The method of claim 6, wherein, additionally in step (2), C6 or lower linear alpha olefin is separated from the resulting reaction mixture of step (1).

10. The method of claim 6, wherein the C6 or lower linear alpha olefin separated in step (2) is distilled additionally.

11. The method of claim 6, wherein the mixture distilled in the dividing wall column in step (3) comprises, based on total 100 % by weight of the mixture, 50 to 95 % by weight of the solvent having a boiling point lower than C8 linear alpha olefin, 4 to 40 % by weight of the C8 linear alpha olefin, and 0.5 to 10 % by weight of the C10 or higher linear alpha olefin.

12. The method of claim 6, wherein the distillation in the dividing wall column in step (3) is conducted at a temperature of 40°C to 250°C and under a pressure of 0.1 bar to 15 bar.

13. The method of claim 6, wherein the solvent discharged from the dividing wall column in step (3) is recovered for reuse in the oligomerization reaction of ethylene in step (1).

14. The method of claim 6, wherein the C10 or higher linear alpha olefin discharged from the dividing wall column in step (3) is distilled additionally.

15. A device for preparing ethylene oligomer, the device comprising:
(a) an ethylene oligomerization reactor;
(b) a first distillation column in fluid communication with the bottom of the ethylene oligomerization reactor; and
(c) a dividing wall column in fluid communication with the bottom of the first distillation column,
wherein the device is operated in a manner that:
in the ethylene oligomerization reactor, ethylene oligomerization reaction is conducted in a solvent having a boiling point lower than C8 linear alpha olefin,
the resulting mixture of the ethylene oligomerization reaction is discharged from the bottom of the ethylene oligomerization reactor, and it is then fed into the first distillation column and distilled therein,
as a result of the distillation in the first distillation column, a mixture comprising the solvent and C8 or higher linear alpha olefins is discharged from the bottom of the first distillation column, and it is then fed into the dividing wall column and distilled therein, and
as a result of the distillation in the dividing wall column, the solvent is discharged from the overhead of the dividing wall column, C8 linear alpha olefin is discharged from the first side of the dividing wall column, and C10 or higher linear alpha olefin is discharged from the bottom or a lower side than the first side of the dividing wall column.

16. The device of claim 15, further comprising an ethylene recovery line fluidly communicating the first distillation column and the ethylene oligomerization reactor.

17. The device of claim 15, further comprising a device for distilling C6 or lower linear alpha olefin which is in fluid communication with the overhead of the first distillation column.

18. The device of claim 15, further comprising a solvent recovery line fluidly communicating the dividing wall column and the ethylene oligomerization reactor.

19. The device of claim 15, further comprising a device for distilling C10 or higher linear alpha olefin which is in fluid communication with the bottom of the dividing wall column.
